# EUROPÄISCHE PATENTSCHRIFT

(11) **EP 0 645 167 B1**
(45) Veröffentlichungstag und Bekanntmachung des Hinweises auf die Patenterteilung: **18.12.1996**
(21) Anmeldenummer: 94113049.4
(22) Anmeldetag: 22.08.1994
(51) Int. Cl.: B01D 11/02, G01N 30/06

(54) **Verfahren zur Gewinnung von pharmakologisch wirksamen Verbindungen aus komplexen Stoffgemischen**
Process for obtaining pharmacological active compounds from complex substance mixtures
Procédé d'obtention de composés pharmacologiques actifs à partir de mélanges complexes de substances

(30) Priorität: 27.08.1993 DE 4328799
(43) Veröffentlichungstag der Anmeldung: 29.03.1995
(73) Patentinhaber: HOECHST AKTIENGESELLSCHAFT, 65929 Frankfurt am Main (DE)
(72) Erfinder: Haas, Peter, Dr., D-66113 Saarbrücken (DE); Engelhardt, Heinz, Prof. Dr., D-66129 Saarbrücken (DE); Müllner, Stefan, Dr., D-65239 Hochheim (DE)

(56) Entgegenhaltungen:
- ANALYTICAL CHEMISTRY, Bd.63, Nr.19, 1. Oktober 1991, WASHINGTON, DC, US Seiten 2152 - 2155 J.W.HILLS ET AL. 'Simultaneous Supercritical Fluid Derivatization and Extraction'
- ANALYTICAL BIOCHEMISTRY, Bd.200, Nr.1, Januar 1992, NEW YORK, US Seiten 7 - 19 L.Q.XIE ET AL. 'Biomedical Applications of Analytical Supercritical Fluid Separation Techniques'
- J. OF LIPID RESEARCH, Bd.24, Nr.8, August 1983 Seiten 1085 - 1100 K.D.R.SETCHELL ET AL. 'General methods for the analysis of metabolic profiles of bile acids and related compounds in feces'

## Beschreibung

Der Nachweis und die Wiederfindung von pharmakologisch wirksamen Verbindungen und deren Metaboliten in biologischen Matrices bereitet oft große Probleme, da Extraktionen mit organischen Lösungsmitteln oder Lösungsmittelgemischen und/oder naßchemische Verfahren in der Regel zeitraubend und nicht quantitativ sind. Zusätzlich ist der meist nicht zu vernachlässigende Verbrauch an organischen Lösungsmitteln zu berücksichtigen. Zudem kann die Probenvorbereitungsmethode in den meisten Fällen nicht on-line mit der Analysemethode gekoppelt werden.

Die genannten Nachteile verhindern die verbreitete Anwendung solcher Methoden in der klinischen Diagnostik durch das Fehlen einer einfachen und reproduzierbaren Analysenmethode. Das vorrangige Problem ist dabei weniger die Trennung als vielmehr die aufwendige und daher wenig reproduzierbare Probenvorbereitung. Verbesserungen können nur bei Einsatz selektiver Extraktionsverfahren erzielt werden.

Für die Probenvorbereitung kommt die Extraktion mit überkritischen Fluiden (SFE) als Alternative in Betracht, da sie die oben genannten Nachteile nicht besitzt. Meist sind allerdings die interessanten Metabolite zu polar für eine direkte Extraktion mit SFE, oder die Isolierung aus komplexen Stoffgemischen erfordert einen besonders hohen Zeitaufwand (K.D.R. Setchell et al., J. of Lipid Research, Vol. 24, (1983), Seiten 1085-1100). Arzneimittel und Xenobiotika werden in der Regel im Körper durch die Leber in polarere Metabolite umgewandelt - z.B. in Alkohole, Carbonsäuren, Glukoronide oder Sulfatester - , um eine Ausscheidung zu erleichtern.

Aufgabe der vorliegenden Erfindung war es, ein Verfahren zur Extraktion und Analyse von freien organischen Säuren und Hydroxysäuren, Fettalkoholen, Zuckern und hydroxysubstituierten Steroiden und Ester der vorgenannten Verbindungen aus komplexen Stoffgemischen zu entwickeln, bei dem die Extraktion im wesentlichen vollständig ist.

Es wurde nun ein Verfahren gefunden, durch das freie organische Säuren und Hydroxysäuren, Fettalkohole, Zucker und hydroxy-substituierte Steroide und deren Ester im wesentlichen vollständig aus komplexen Stoffgemischen gewonnen werden können, das dadurch gekennzeichnet ist, daß man
A) das zu extrahierende Material mit einem polaren Lösungsmittel oder einem Gemisch polarer Lösungsmittel vermischt und das erhaltene Gemisch zur Trockne einengt,
B) das aus A) erhaltene Material silyliert, verestert oder acyliert und
C) das aus B) erhaltene Material mit einem überkritischen Fluid bei einem Druck von mehr als 200 bar extrahiert.

Komplexe Stoffgemische sind z.B. Urin, Organgewebe, Feces oder Serum von Tieren oder Menschen, die mechanisch zerkleinert, lyophilisiert oder extrahiert sein können.

Geeignete Gemische polarer Lösungsmittel sind z.B. Methanol/Wasser im Volumenverhältnis 2:1, Methanol/Wasser/Heptan im Volumenverhältnis 1:1:0,5 oder Lösungsmittelmischungen mit vergleichbarer Polarität.

Die Art der Derivatisierung richtet sich im wesentlichen nach der verwendeten Analysemethode nach der Extraktion. Bei der Analyse mit Hilfe der Gaschromatographie (GC) können die Methoden der Silylierung, z.B. mit Hexamethyldisilazan (HMDS) und Trimethylchlorsilan (TMCS), oder der Veresterung, z.B. mit Bortrifluorid in Methanol, oder der Acylierung, z.B. mit p-Dimethylaminopyridin/Acetanhydrid, angewendet werden.

Der Begriff überkritisches Fluid steht z.B. für CO₂, N₂O, n-Butan, n-Pentan oder C₁-Fluorchlorkohlenwasserstoffe, vorzugsweise für CO₂.

Als erfindungsgemäß extrahierbare Verbindungen kommen in Betracht:
A) freie organische Säuren,
   beispielsweise aromatische oder aliphatische Carbonsäuren wie Capronsäure, Valeriansäure, Adipinsäure, Glutarsäure, Indolcarbonsäure oder β-Phenylpropansäure,
   insbesondere gesättigte oder ungesättigte Fettsäuren, vorzugsweise höhere Fettsäuren mit 12 bis 18 C-Atomen wie Caprin-, Laurin-, Myristin-, Palmitin-oder Stearinsäure, vorzugsweise Myristin-, Palmitin-, Stearin-, Öl- und Linolsäure;
B) Hydroxysäuren,
   beispielsweise niedere Mono-, Di- oder Polyhydroxy-carbonsäuren wie Milch-, Äpfel-, Wein- oder Citronensäure, aber auch Hydroxyfettsäuren wie Ricinolsäure oder aromatische Säuren wie Salicylsäure;
C) Fettalkohole, die gesättigt oder ungesättigt sein können,
   z.B. solche mit 12 bis 18 C-Atomen, beispielsweise Myristyl-, Cetyl-, Stearyl- oder Oleylalkohol;
D) Zucker,
   z.B. Monosaccharide, beispielsweise Glucose, Mannose, Fructose oder Disaccharide, beispielsweise Saccharose oder Lactose, wobei die Monosaccharide bevorzugt sind;
E) Hydroxy-substituierte Steroide,
   beispielsweise Cholsäure, Chenodesoxycholsäure, Desoxycholsäure, Ursodesoxycholsäure, Lithocholsäure, Pregnantriol, Cholesterin, Cholesterinester, z.B. Coprosterin, β-Sitosterin, insbesondere jedoch die Gallensäuren wie Cholsäure, Lithocholsäure, Desoxycholsäure und Ursodesoxycholsäure.

Als erfindungsgemäß extrahierbare Ester seien beispielsweise genannt solche mit niederen Alkoholen, insbesondere der Methylester, sowie die durch Derivatisierung erhaltenen Silylester, vorzugsweise der durch Trimethylchlorsilan erhaltene TMS-ester.

Von den vorstehend genannten Verbindungen sind erfindungsgemäß besonders bevorzugt die höheren Fettsäuren, Monosaccharide, Gallensäuren und Cholesterin, insbesondere die Gallensäuren.

Bislang stellte die Extraktion von Gallensäuren aus biologischen Matrices ein großes Problem dar, weil Gallensäuren neben ihrem amphiphilen Verhalten, ihrer hohen Polarität und ihrer Tendenz zur Selbstassoziation noch hohe Bindungsaffinität zu Serumproteinen oder zu bestimmten Polysacchariden aus Pflanzenfasern besitzen. Diese Eigenschaften der Gallensäuren erschweren die Extraktion aus Feces bzw. Serum mit herkömmlichen Methoden. Diese Extraktionsprobleme konnten erfindungsgemäß überwunden werden.

Beim Verfahrensschritt A) geht man wie folgt vor:

Das zu analysierende Material, beispielsweise Rattenkot, wird mit einem Lösungsmittelgemisch, z.B. Methanol/Wasser im Volumenverhältnis 2:1, vermischt und bei Raumtemperatur gerührt. Grobes Material wird gegebenenfalls vor oder nach dem Rühren im Methanol/Wasser-Gemisch bis zu einer Partikelgröße von etwa 0,2 mm zerkleinert. Für die Analyse von Cholesterinestern, Fettsäuren oder Fettalkoholen ist der Zusatz von einem mit polaren Lösemitteln nicht mischbaren Lösemittel, z.B. n-Heptan günstig. Anschließend wird das Material unter vermindertem Druck, vorzugsweise bis zur Trockne, eingeengt.

Das aus Verfahrensschritt A) erhaltene Material wird im Verfahrensschritt B) nach bekannten Methoden derivatisiert. Wenn das Material mit Hilfe der Gaschromatographie analysiert werden soll, erfolgt die Derivatisierung durch Silylierung oder Acylierung.

Bei der Silylierung wird das aus A) stammende Material z.B. in Pyridin aufgenommen und mit Hexamethyldisilazan (HMDS) und Trimethylchlorsilan (TMCS) für etwa 15 min bei 25°C gerührt und dann unter vermindertem Druck eingeengt bis das Material rieselfähig ist. Dabei sollte bei Temperaturen von weniger als 50°C gearbeitet werden.

Sowohl bei einer Veresterung als auch bei der Acylierung sollten Methoden verwendet werden, die ohne Säurekatalyse auskommen, wie z.B. Bortrifluorid in Methanol. Anschließend kann wie oben gezeigt silyliert oder beispielsweise mit p-Dimethylaminopyridin/Acetanhydrid acyliert werden.

Das aus Verfahrensschritt B) erhaltene Material wird mit einem überkritischen Fluid extrahiert. Bevorzugtes Fluid ist CO₂, das bei einem Druck von mehr als 200 bar, bevorzugt 430 bis 480 bar eingesetzt wird. Die Extraktion erfolgt bei Temperaturen von 30 bis 50°C, bevorzugt 35 bis 40°C für 1 bis 4 Stunden, bevorzugt 2 bis 3 Stunden. Gegebenenfalls können dem Kohlendioxid noch 5 bis 10 % polare Zusätze, wie z.B. Methanol, Ethanol oder Aceton, oder während der Extraktion n-Heptan zugefügt werden.

Der erhaltene Extrakt wird nach bekannten Methoden für die GC-Analyse vorbereitet. Bei der Silylierung hat sich eine Nachbehandlung des Extraktes mit Pyridin, HMDS und TMCS bewährt.

Der Aufbau und die Funktionsweise der SFE-Apparatur sind einfach. Flüssiges CO₂ unter dem für die jeweilige Extraktion erforderlichen Druck wird mit einer Pumpe- hierfür können sowohl HPLC-Pumpen, als auch Langhubkolbenpumpen verwendet werden - durch die in einem auf die Extraktionstemperatur geheizten Ofen befindlichen Extraktionskammer gepumpt, welche die Probe enthält. Dabei geht das Extraktionsmittel erst bei Eintritt in den Ofen in den überkritischen Zustand über. Hinter der Extraktionskammer befindet sich ein Restriktor, der als Strömungswiderstand die Aufgabe hat, bei mäßig hohen Flußraten den Extraktionsdruck in der Kammer aufrechtzuerhalten.

Das Ende des Restriktors wird aus dem Ofen herausgeführt und in ein Auffanggefäß getaucht, welches ein für die zu extrahierenden Analyten geeignetes Lösemittel enthält. Bei der Extraktion leicht flüchtiger Komponenten ist eine Kühlung dieser Vorlage sinnvoll.

Im Folgenden wird eine detaillierte Beschreibung der zur Durchführung des erfindungsgemäßen Verfahrens eingesetzten SFE- und GC-Apparaturen gegeben.
- SFE:: Modell SFE/50, Suprex (Pittsburgh, PA, USA)
als Extraktionskammer dienen Stahlrohre (12 cm x 7 mm i.D.), die mit Swagelok-Fittings (1/16 Zoll-Ausgänge) verschraubt sind,
n-Heptan wurde mit Hilfe einer HPLC-Pumpe Modell 420, Kontron (München, D) über ein vor der Kammer befindliches 1/16 Zoll-T-Stück zugemischt,
als Restriktoren werden Quarzglaskapillaren (8 m x 75 µm i.D.),
Microquartz (München, D) verwendet,
als Vorlage dient ein 10 ml Langhalsmeßkolben, der mit etwa 5 ml n-Heptan gefüllt ist,
das Kohlendioxid der Reinheit 4,5 ist von Messer Griesheim (Saarbrücken, D) bezogen worden.
- GC:: Modell HRGC 5300, Carlo Erba (Mailand, I)
Säule: DB-5, 15 m x 250 µm i.D., df = 0,25 µm, J & W Scientific (Folsom, CA, USA)
Injektion: 1 µl, Split 20:1; Trägergas: N₂, 42,3 cm³/s; Detektion: FID

Die folgenden Retentionsbereiche und Retentionszeiten wurden für erfindungsgemäß extrahierte Verbindungen der vorstehend beschriebenen Säule unter den dort genannten Bedingungen und folgendem Temperaturprogramm ermittelt: 2 min bei 50°C, mit 20°C/min auf 170°C, mit 10°C/min auf 280°C, mit 2°C/min auf 310°C, 5 min bei 310°C. Genauigkeit der Retentionszeiten beträgt etwa ± 0,05 min.

| Verbindungsklasse | Retentionsbereich/min |
|---|---|
| Silane, Siloxane | 2 - 8 |
| Monosaccharide | 8 - 15 |
| Fettsäuren und ihre TMS-Ester | 15 - 19 |
| Steroide, allgemein | 26 - 34 |
| Gallensäuren, speziell | 30 - 33 |
| Cholesterinester | > 36 |

| Verbindung | Retentionszeit/min |
|---|---|
| β-Phenylpropansäure | 8,6 |
| β-Phenylpropansäure, TMS-Ester | 10,35 |
| Myristinsäure, TMS-Ester | 14,4 |
| Indolcarbonsäure, TMS-Ester | 15,15 |
| Myristinsäure | 15,65 |
| Palmitinsäure, TMS-Ester | 16,4 |
| Palmitinsäure | 17,5 |
| Linolsäure, TMS-Ester | 18,0 |
| Ölsäure, TMS-Ester | 18,05 |
| Stearinsäure, TMS-Ester | 18,2 |
| Stearinsäure | 19,2 |
| Coprosterin (TMS) | 27,7 |
| Cholesterin | 28,6 |
| Cholesterin (TMS) | 29,25 |
| Lithocholsäure (TMS)₄ | 31,0 |
| β-Sitosterin | 31,3 |
| Desoxycholsäure (TMS)₄ | 31,95 |
| Cholsäure (TMS)₄ | 32,3 |
| Ursodesoxycholsäure (TMS)₄ | 32,7 |
| β-Sitosterin (TMS) | 32,75 |

Die einzelnen Schritte der Methode sind in der folgenden Tabelle nochmals zusammengestellt. Während die Extraktion und nachfolgende Reinigung der Extrakte bei herkömmlichen Methoden wenigstens 2 Tage erfordert (K.D.R. Setchell et al., J. Lipid Research, Vol. 24, (1983), Seiten 1085 - 1100) entfallen bei dem hier vorliegenden Verfahren auf die gesamte Probenvorbereitung etwa 4 Stunden.

| Arbeitsschritt | Durchführung | Materialien | Dauer |
|---|---|---|---|
| Aktivierung | Rühren bei Raumtemperatur, Einrotieren | 30 ml Methanol | 45 min |
| Derivatisierung | Rühren bei Raumtemperatur, Einrotieren | 20 ml Pyridin, 2,5 ml HMDS, 0,8 ml TMCS | 45 min |
| Extraktion | Füllen der Extraktionskammer, Extraktion | 80 L CO₂(g), 15 ml n-Heptan | 2,5 h |

In dem folgenden Beispiel wird das erfindungsgemäße Verfahren im einzelnen beschrieben.

### Beispiel

1 g getrockneter Rattenkot wird auf eine Partikelgröße von etwa 0,2 mm zerkleinert und in 20 ml Methanol/Wasser (2:1) bei 25°C 15 min gerührt. Anschließend wird das Gemisch unter vermindertem Druck zur Trockne eingeengt. Das erhaltene Material wird mit 15 ml Pyridin, 2,5 ml Hexamethyldisilazan (HMDS) und 0,8 ml Trimethylchlorsilan (TMCS) versetzt, 15 min bei 25°C gerührt und anschließend bei 40°C unter vermindertem Druck zur Trockne eingeengt. Das erhaltene Material wird 2 Stunden lang mit CO₂ bei 40°C und 450 bar extrahiert (SFE-Extraktionsgerät, Modell SFE/50, Suprex (Pittsburgh, PA., USA, wie oben beschrieben)). Als Vorlage dienen etwa 5 ml n-Heptan in einem 10 ml Kolben. Der erhaltene Extrakt wird getrocknet und mit 250 µl Pyridin, 150 µl HMDS und 50 µl TMCS versetzt. Nach 10 min kann eine Probe im Gaschromatographen (Modell HRGC 5300, Carlo Erba, Mailand) analysiert werden. Eine 1-µl-Probe wird auf die oben beschriebene Säule gespritzt. Anschließend wird folgendes Temperaturprogramm durchgeführt:

2 min bei 50°C, mit 20°C/min auf 170°C, mit 10°C/min auf 280°C, mit 2°C/min auf 310°C, 5 min bei 310°C.

Das erhaltene Chromatogramm zeigt die vollständige Extraktion von β-Phenylpropansäure; β-Phenylpropansaure-TMS-Ester; Myristinsäure-TMS-Ester; Myristinsäure; Palmitinsäure-TMS-Ester; Palmitinsäure; Linolsäure-TMS-Ester; Ölsäure-TMS-Ester; Stearinsäure-TMS-Ester; TMS-Coprosterin; TMS-Cholesterin; Lithocholsäure (TMS)₄; β-Sitosterin; Desoxycholsäure (TMS)₄ und TMS-β-Sitosterin.

Die Identität der genannten Verbindungen wird mittels Gaschromatographie und Massenspektroskopie sichergestellt. Die Vollständigkeit der Extraktion wird in einem weiteren Chromatogramm bewiesen.

## Patentansprüche

1. Verfahren zur Gewinnung von freien organischen Säuren, Hydroxysäuren, Fettalkoholen, Zuckern und hydroxysubstituierten Steroiden und Estern der vorgenannten Verbindungen aus komplexen Stoffgemischen, dadurch gekennzeichnet, daß man
A) das zu extrahierende Material mit einem polaren Lösungsmittel oder einem Gemisch polarer Lösungsmittel vermischt und das erhaltene Gemisch zur Trockne einengt,
B) das aus A) erhaltene Material silyliert, verestert oder acyliert und
C) das aus B) erhaltene Material mit einem überkritischen Fluid bei einem Druck von mehr als 200 bar extrahiert.

2. Verfahren gemäß Anspruch 1, dadurch gekennzeichnet, daß man als überkritisches Fluid CO₂ einsetzt.

3. Verfahren gemäß Anspruch 1, dadurch gekennzeichnet, daß man als polares Lösungsmittel ein Methanol-Wasser-Gemisch im Volumenverhältnis 2:1 einsetzt.

4. Verfahren gemäß Anspruch 1, dadurch gekennzeichnet, daß man bei einem Druck von 430 bis 480 bar extrahiert.

5. Verfahren gemäß Anspruch 1, dadurch gekennzeichnet, daß man Fettsäuren, Monosaccharide, Cholesterin oder Gallensäuren extrahiert.

6. Verfahren gemäß Anspruch 1, dadurch gekennzeichnet, daß man β-Phenylpropansäure, Myristinsäure, Indolcarbonsäure, Palmitinsäure, Linolsäure, Ölsäure, Stearinsäure, Coprosterin, Cholesterin, Lithocholsäure, β-Sitosterin, Desoxycholsäure, Cholsäure, Ursodesoxycholsäure oder Ester der vorgenannten Verbindungen sowie Mischungen der Verbindungen extrahiert.

7. Verfahren gemäß Anspruch 1, dadurch gekennzeichnet, daß man mit Hexamethyldisilazan oder Trimethylchlorsilan silyliert, mit Bortrifluorid in Methanol verestert oder mit p-Dimethylaminopyridin/Acetanhydrid acyliert.

## Claims

1. A process for obtaining free organic acids, hydroxy acids, fatty alcohols, sugars and hydroxy-substituted steroids and esters of the aforementioned compounds from complex mixtures of substances, which comprises
A) mixing the material to be extracted with a polar solvent or a mixture of polar solvents, and concentrating the resulting mixture to dryness,
B) silylating, esterifying or acylating the material obtained from A) and
C) extracting the material obtained from B) with a supercritical fluid under a pressure of more than 200 bar.

2. The process as claimed in claim 1, wherein CO₂ is used as supercritical fluid.

3. The process as claimed in claim 1, wherein a methanol/water mixture in the ratio 2:1 by volume is used as polar solvent.

4. The process as claimed in claim 1, wherein extraction is carried out under a pressure of 430 to 480 bar.

5. The process as claimed in claim 1, wherein fatty acids, monosaccharides, cholesterol or bile acids are extracted.

6. The process as claimed in claim 1, wherein β-phenylpropanoic acid, myristic acid, indolecarboxylic acid, palmitic acid, linoleic acid, oleic acid, stearic acid, coprosterol, cholesterol, lithocholic acid, β-sitosterol, deoxycholic acid, cholic acid, ursodeoxycholic acid or esters of the aforementioned compounds as well as mixtures of the compounds are extracted.

7. The process as claimed in claim 1, wherein silylation is carried out with hexamethyldisilazane or trimethylchlorosilane, esterification is carried out with boron trifluoride in methanol, or acylation is carried out with p-dimethylaminopyridine/acetic anhydride.

## Revendications

1. Procédé pour l'obtention d'acides organiques libres, d'acides hydroxylés, d'alcools gras, de sucres et de stéroïdes substitués par des groupes hydroxy, et d'esters des composés précités, à partir de mélanges complexes de substances, caractérisé en ce que
A) on mélange la substance à extraire avec un solvant polaire ou un mélange de solvants polaires, et le mélange obtenu est concentré à siccité,
B) le produit obtenu en A) est silylé, estérifié ou acylé, et
C) le produit obtenu en B) est extrait avec un fluide supercritique, sous une pression de plus de 200 bars.

2. Procédé selon la revendication 1, caractérisé en ce que l'on utilise comme fluide supercritique CO₂.

3. Procédé selon la revendication 1, caractérisé en ce que l'on utilise comme solvant polaire un mélange de méthanol et d'eau dans le rapport 2:1 en volume.

4. Procédé selon la revendication 1, caractérisé en ce que l'on effectue l'extraction sous une pression de 430 à 480 bars.

5. Procédé selon la revendication 1, caractérisé en ce que l'on extrait des acides gras, des monosaccharides, le cholestérol ou des acides biliaires.

6. Procédé selon la revendication 1, caractérisé en ce que l'on extrait l'acide β-phénylpropionique, l'acide myristique, l'acide indolecarboxylique, l'acide palmitique, l'acide linoléique, l'acide oléique, l'acide stéarique, le coprostérol, le cholestérol, l'acide lithocholique, le β-sitostérol, l'acide désoxycholique, l'acide cholique, l'acide ursodésoxycholique ou des esters des composés précités, ainsi que des mélanges des composés.

7. Procédé selon la revendication 1, caractérisé en ce que l'on effectue une silylation avec de l'hexaméthyldisilazane ou du triméthylchlorosilane, une estérification avec du trifluorure de bore dans du méthanol ou une acylation avec de la p-diméthylaminopyridine/anhydride acétique.
